# EUROPEAN PATENT SPECIFICATION

(11) **EP 4 358 022 B1**
(45) Date of publication and mention of the grant of the patent: **17.12.2025**
(21) Application number: 22824751.6
(22) Date of filing: 24.05.2022
(51) Int. Cl.: G06T 7/00, A61B 6/03, G16H 50/20

(54) **MEDICAL IMAGE DIAGNOSTIC SYSTEM, MEDICAL IMAGE DIAGNOSTIC METHOD, AND PROGRAM**
DIAGNOSESYSTEM FÜR MEDIZINISCHE BILDER, DIAGNOSEVERFAHREN FÜR MEDIZINISCHE BILDER UND PROGRAMM
SYSTÈME DE DIAGNOSTIC D'IMAGE MÉDICALE, PROCÉDÉ DE DIAGNOSTIC D'IMAGE MÉDICALE ET PROGRAMME

(30) Priority: 17.06.2021 JP 2021100611
(43) Date of publication of application: 24.04.2024
(73) Proprietor: FUJIFILM Corporation, Tokyo 106-8620 (JP)
(72) Inventor: MORITA, Masaharu, Tokyo 106-8620 (JP); MASUMOTO, Jun, Tokyo 106-8620 (JP)
(74) Representative: Dehns Germany Partnerschaft mbB
(86) International application number: PCT/JP2022/021224
(87) International publication number: WO 2022/264757

(56) References cited:
- JP-A- 2012 026 982
- JP-A- 2019 067 069
- JP-B1- 6 885 517
- JP-B1- 6 885 517
- US-A1- 2019 125 306
- US-A1- 2020 202 103
- US-A1- 2020 202 103

## Description

### BACKGROUND OF THE INVENTION

### 1. Field of the Invention

The present invention relates to a medical image diagnosis system, a medical image diagnosis method, and a program, and particularly, to a technology for supporting diagnosis of a medical image.

### 2. Description of the Related Art

A system that finds and supports diagnosis of an abnormal region in a medical image by using artificial intelligence (AI) is known.

For example, JP2006-340835A discloses a medical image processing system that provides only detection information on an abnormal shadow candidate suspected to be a true positive abnormal shadow and/or an abnormal shadow candidate with low visibility regarding the abnormal shadow candidates detected from the medical images, and that prevents oversight of doctors and improves the efficiency of image interpretation work. JP6885517B1 discloses a diagnostic support device and a model generation device for evaluating medical images. US2019/0125306A1 discloses a medical image transmitting method that includes a step of determining whether an object has an abnormality. US2020/0202103A1 discloses apparatus and method for detecting features indicative of diabetic retinopathy in retinal images.

### SUMMARY OF THE INVENTION

For health management, a health checkup is performed to examine a health state of a subject. In the health checkup, the subject is mainly a healthy person, and the purpose of diagnosis, a target organ, and a target illness are limited. For regions with possible abnormalities in medical images obtained by the health checkup, it is necessary for a doctor to determine whether there are indeed abnormalities, diagnose possible disease names, and create detailed reports, and these tasks are important.

On the other hand, the doctor also has to confirm the image with no abnormal region. For example, in a case of a health checkup mainly for young people, since a patient with an abnormality is unlikely to be present, a doctor has to confirm a large number of "images with no abnormalities", which is a heavy burden on the doctor.

In contrast, it is conceivable to support image diagnosis by using lesion detection AI. However, the lesion detection AI is usually created for each illness, and since the types of diseases are enormous, it is difficult to create AI corresponding to all diseases. In addition, the amount of training data is small for rare diseases, and it is difficult to create lesion detection AI. Furthermore, lesion detection AI cannot be created for an unknown disease, but a doctor is responsible for diagnosing the unknown disease. As described above, even in a case in which the number of lesion detection AIs is increased and the accuracy of each lesion detection AI is increased, there is a problem that the output accuracy of the lesion detection AI is limited.

In addition, the line-up of lesion detection AI is enormous in a case of considering the number of target illnesses and the number of manufacturers. Therefore, there is a problem that it is difficult to perform all types of existing lesion detection AI processing on all input images in terms of hardware resources and processing time.

The present invention has been made in view of such circumstances, and an object of the present invention is to provide a medical image diagnosis system, a medical image diagnosis method, and a program that reduce a burden on a doctor in a case of performing image diagnosis on a large number of medical images, such as a health checkup.

According to a first aspect of the present invention, a medical image diagnosis apparatus is provided as claimed in claim 1.

A case in which the medical image is normal is, for example, a case in which the medical image can be said to be an image of a healthy person. The healthy person refers to a person who is healthy, for example, a person who does not have a disease, an illness, or a lesion. According to the present aspect, it is possible to reduce the burden on the doctors in a case in which the image diagnosis is performed on a large number of medical images.

It is preferable that the at least one processor performs third determination of determining the presence or absence of the abnormality from the medical image in a case in which it is determined that the medical image is normal in the first determination.

It is preferable that the at least one processor performs the third determination at a timing different from a timing of the second determination.

It is preferable that the at least one processor performs the first determination with a probability that the medical image is normal, and performs the third determination in a case in which the medical image is determined to be normal with a probability lower than a first threshold value in the first determination.

It is preferable that the at least one processor performs the first determination by using a first trained model that outputs whether or not the medical image is normal in a case in which the medical image is input, and retrains the first trained model by using a medical image which is determined to have the abnormality in the third determination.

It is preferable that the first trained model is a trained model that has been trained by using combinations of an abnormal medical image, a normal medical image, and labels indicating the presence or absence of the abnormality, as a training data set.

It is preferable that, for a first case in which it is determined that the medical image is normal in the first determination, a second case in which it is determined that the medical image is not normal in the first determination and it is determined that the abnormality is present in the second determination, and a third case in which it is determined that the medical image is not normal in the first determination and it is determined that the abnormality is absent in the second determination, the at least one processor displays a determination result of the medical image on a display differently for the second and third cases than for the first case.

It is preferable that the at least one processor displays the determination result of the medical image on the display differently between the second case and the third case.

It is preferable that the at least one processor performs different types of post-processing on the medical image for the second and third cases than for the first case.

It is preferable that the at least one processor performs the first determination and the second determination for each organ of the subject from the medical image.

It is preferable that the at least one processor performs the second determination by using a second trained model that outputs the abnormality of the medical image in a case in which the medical image is input.

According to a second aspect of the present invention, a medical image diagnosis method for achieving the object described above is provided as claimed in claim 12. According to the second aspect, it is possible to reduce the burden on the doctors in a case in which the image diagnosis is performed on a large number of medical images.

According to a third aspect of the present invention, a computer-readable non-transitory storage medium is provided as claimed in claim 13.

According to the present invention, it is possible to reduce the burden on the doctors in a case in which the image diagnosis is performed on a large number of medical images.

### BRIEF DESCRIPTION OF THE DRAWINGS

Fig. 1 is a block diagram illustrating a medical image diagnosis system according to the present embodiment.
Fig. 2 is a flowchart illustrating a medical image diagnosis method.
Fig. 3 is a process diagram illustrating the medical image diagnosis method.
Fig. 4 is a diagram illustrating a display form.
Fig. 5 is a diagram illustrating a display form.
Fig. 6 is a diagram illustrating a display form.
Fig. 7 is a diagram illustrating a display form.

### DESCRIPTION OF THE PREFERRED EMBODIMENTS

Hereinafter, a preferred embodiment of the present invention will be described in accordance with the accompanying drawings.

### [Configuration of Medical Image Diagnosis System (Medical Image Diagnosis Support System)]

The medical image diagnosis system according to the present embodiment reduces the burden on the doctor in a case of determining the presence or absence of an abnormality in a large amount of medical images such as a health checkup, and determining whether or not the medical image is normal (whether or not it is an image of a healthy person).

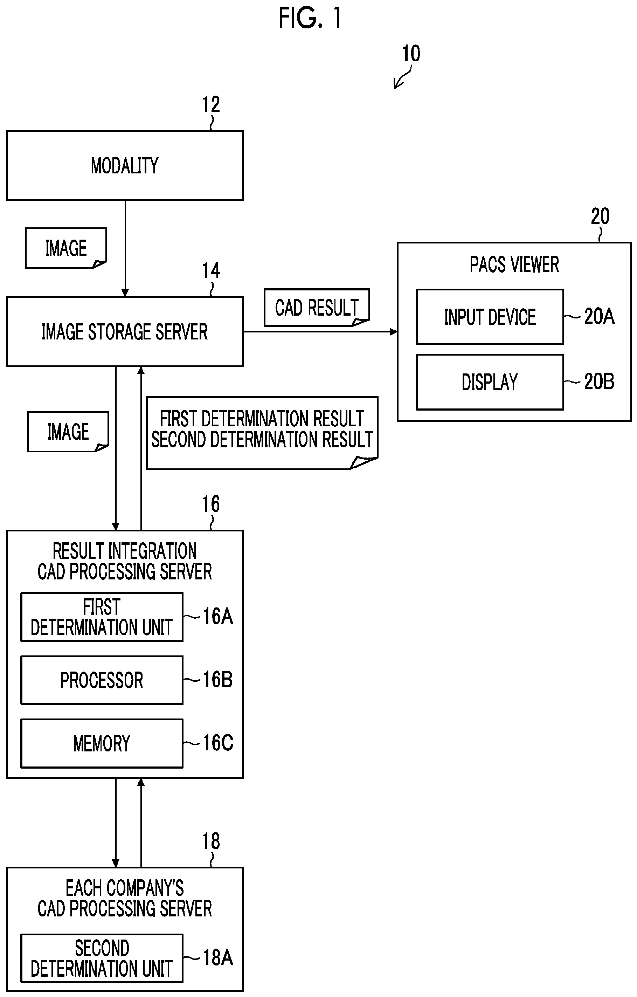

Fig. 1 is a block diagram illustrating a medical image diagnosis system 10 according to the present embodiment. As illustrated in Fig. 1, the medical image diagnosis system 10 comprises a modality 12, an image storage server 14, each company's computer-aided diagnosis (CAD) processing server 18, a result integration CAD processing server 16, and a picture archiving and communication system (PACS) viewer 20.

The modality 12, the image storage server 14, each company's CAD processing server 18, the result integration CAD processing server 16, and the PACS viewer 20 are each connected to a communication network such as the Internet so that the data can be transmitted and received.

The modality 12 is an imaging apparatus that images an examination target part of a subject and generates a medical image. The modality 12 includes, for example, at least one of an X-ray imaging apparatus, a computed tomography (CT) apparatus, a magnetic resonance imaging (MRI) apparatus, a positron emission tomography (PET) apparatus, an ultrasound apparatus, or a computed radiography (CR) apparatus using a planar X-ray detector.

The image storage server 14 is a server that manages the medical image captured by the modality 12. A computer comprising a large-capacity storage device is applied to the image storage server 14. Software providing a function of a database storage system is incorporated in the computer. The image storage server 14 acquires a medical image captured by the modality 12 and stores the medical image in the large-capacity storage device.

The digital imaging and communications in medicine (DICOM) standard can be applied to the format of the medical image. DICOM tag information defined by the DICOM standard may be added to the medical image. The term "image" in the present specification can include the meaning of image data, which is a signal representing an image, in addition to the meaning of an image itself such as a photograph.

The result integration CAD processing server 16 includes a first determination unit 16A. For the medical image acquired from the image storage server 14, the first determination unit 16A performs first determination of determining whether or not the medical image is normal for each organ. Whether or not the medical image is normal is, for example, whether or not the medical image can be said to be an image of a healthy person. The healthy person refers to a person who is healthy, for example, a person who does not have a disease, an illness, or a lesion. The first determination result of the first determination unit 16A is associated with the medical image in the image storage server 14 and is stored in the large-capacity storage device.

A personal computer or a workstation is applied to the result integration CAD processing server 16. The result integration CAD processing server 16 comprises a processor 16B and a memory 16C. The processor 16B performs a command stored in the memory 16C.

A hardware structure of the processor 16B includes various processors to be described below. The various processors include a central processing unit (CPU) that is a general-purpose processor actioning as various functional units by executing software (program), a graphics processing unit (GPU) that is a processor specially designed for image processing, a programmable logic device (PLD) such as a field programmable gate array (FPGA) that is a processor having a circuit configuration changeable after manufacturing, and a dedicated electric circuit or the like such as an application specific integrated circuit (ASIC) that is a processor having a circuit configuration dedicatedly designed to execute a specific type of processing.

One processing unit may be configured by one processor among these various processors, or may be configured by two or more same or different kinds of processors (for example, a combination of a plurality of FPGAs, a combination of the CPU and the FPGA, or a combination of the CPU and GPU). In addition, a plurality of functional units may be formed of one processor. As an example of configuring the plurality of functional units with one processor, first, as represented by a computer such as a client or a server, a form of configuring one processor with a combination of one or more CPUs and software and causing the processor to act as the plurality of functional units is present. Second, as represented by a system on chip (SoC) or the like, a form of using a processor that implements the function of the entire system including the plurality of functional units using one integrated circuit (IC) chip is present. Accordingly, various functional units are configured using one or more of the various processors as a hardware structure.

Furthermore, the hardware structure of the various processors is more specifically an electric circuit (circuitry) in which circuit elements such as semiconductor elements are combined.

The memory 16C stores a command to be executed by the processor 16B. The memory 16C includes a random access memory (RAM) and a read only memory (ROM), which are not illustrated. The processor 16B uses the RAM as a work region to execute software using various programs and parameters including a medical image processing program stored in the ROM, and executes various types of processing of the result integration CAD processing server 16 by using the parameters stored in the ROM.

Each company's CAD processing server 18 is composed of a plurality of CAD processing servers owned by a plurality of companies. Each company's CAD processing server 18 may be a single CAD processing server. Each company's CAD processing server 18 includes a second determination unit 18A. The second determination unit 18A includes a program that performs abnormality detection processing for each organ on the medical image, which is acquired from the image storage server 14 and determined to be abnormal at least by the first determination unit 16A, and that performs second determination to determine the presence or absence of one or more abnormalities from the medical image. The abnormality includes, for example, at least one of a disease, an illness, or a lesion. A second determination result of the second determination unit 18A is associated with the medical image in the image storage server 14 and is stored in the large-capacity storage device.

The second determination unit 18A may be provided in the result integration CAD processing server 16.

The PACS viewer 20 is a terminal device used by a user, such as a doctor, and, for example, a known image viewer for image interpretation is applied. The PACS viewer 20 may be a personal computer, a workstation, or a tablet terminal.

The PACS viewer 20 comprises an input device 20A and a display 20B. The input device 20Aincludes a pointing device, such as a mouse, and an input device, such as a keyboard. The user can input an instruction to the medical image diagnosis system 10 using the input device 20A. The display 20B displays a screen necessary for an operation of the input device 20A, and functions as a part for implementing a graphical user interface (GUI). The medical image captured by the modality 12 is displayed on the display 20B. In addition, the first determination result and the second determination result are displayed on the display 20B as CAD results. A touch panel display in which the input device 20A and the display 20B are integrated may be applied to the PACS viewer 20.

### [Medical Image Diagnosis Method (Medical Image Diagnosis Support Method)]

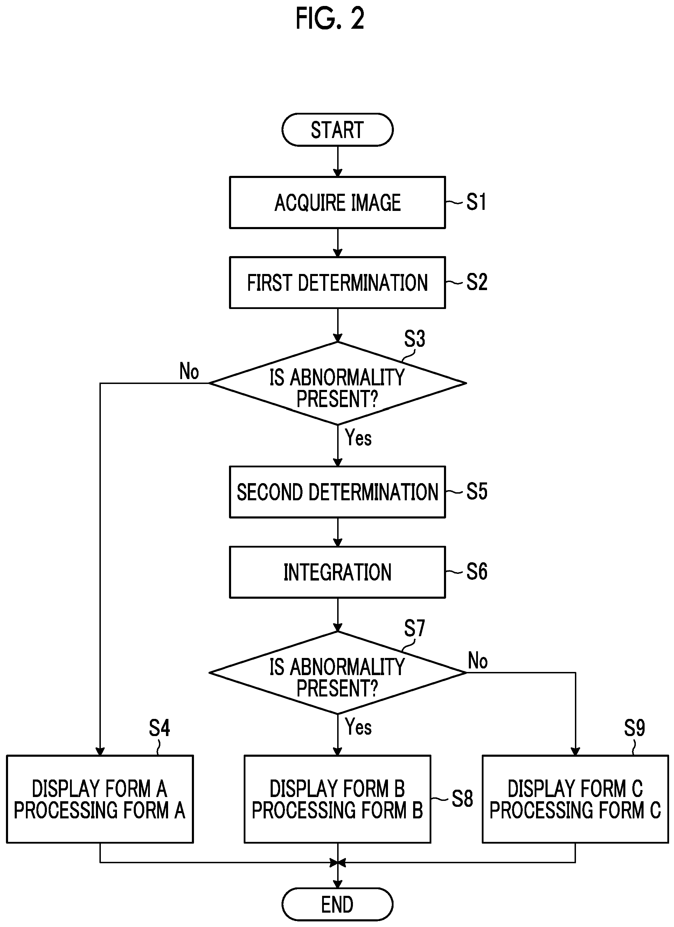

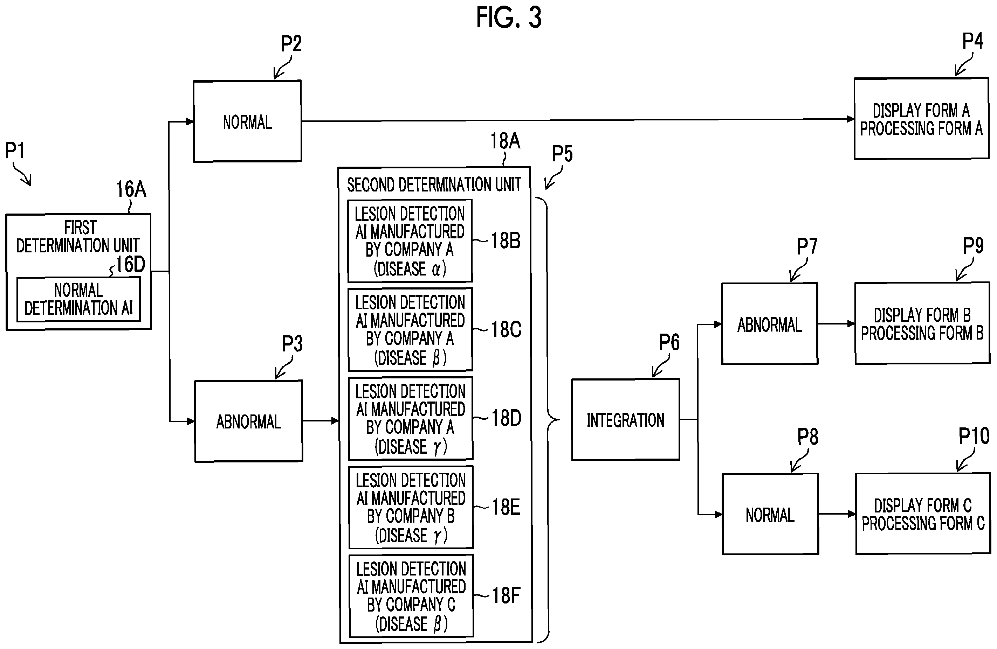

Fig. 2 is a flowchart illustrating a medical image diagnosis method using the medical image diagnosis system 10. In addition, Fig. 3 is a process diagram illustrating the medical image diagnosis method. The medical image diagnosis method is implemented by the processor 16B executing the medical image diagnosis program (medical image diagnosis support program) stored in the memory 16C. In a case in which the second determination unit 18A is included in each company's CAD processing server 18, the method is implemented by a processor (not illustrated) included in each company's CAD processing server 18 executing the medical image diagnosis program stored in the memory (not illustrated) included in each company's CAD processing server 18, and by the processor 16B executing the medical image diagnosis program stored in the memory 16C. The medical image diagnosis program may be provided by a computer-readable non-transitory storage medium. In this case, the result integration CAD processing server 16 may read the medical image diagnosis program from the non-transitory storage medium and store the medical image diagnosis program in the memory 16C.

The medical image diagnosis method is performed for each organ of the subject. Here, as an example, a case of diagnosing a CT image of a lung will be described.

In Step S1, the processor 16B of the result integration CAD processing server 16 causes the image storage server 14 to acquire a CT image of a lung of the subject captured by the modality 12. The image storage server 14 acquires the CT image captured by the modality 12.

In Step S2 (an example of a "first determination step"), the processor 16B inputs the CT image acquired by the image storage server 14 to the first determination unit 16A. As illustrated in Fig. 3, the first determination unit 16A includes a normal determination AI 16D that determines whether or not the medical image is normal.

The normal determination AI 16D is a trained model (an example of a "first trained model") that determines, in a case in which the CT image of the lung is input, whether or not the CT image is normal, and includes a convolutional neural network. The normal determination AI 16D is generated by deep learning using a training data set of a medical image and a label indicating the presence or absence of an abnormality. For example, the normal determination AI 16D is generated by deep learning using a training data set of a normal CT image and a normal label and a training data set of an abnormal CT image and an abnormal label. The normal CT image is a CT image of a healthy person. In addition, the abnormal CT image is a CT image having some abnormality, and is, for example, a CT image of a person having at least one of a disease, an illness, or a lesion.

The normal determination AI 16D is generated to output a degree of normality of the input CT image as a numerical value (a score, an example of a "probability"). The normal determination AI 16D outputs that the CT image is not normal in a case in which the degree of normality of the CT image is less than a predetermined threshold value, and outputs that the CT image is normal in a case in which the degree of normality of the CT image is equal to or greater than the threshold value. The first determination unit 16A inputs the CT image to the normal determination AI 16D and performs the first determination (Process P1).

In Step S3, the processor 16B acquires the first determination result from the first determination unit 16A, and determines whether the CT image is normal from the first determination result. That is, the processor 16B determines the presence or absence of the abnormality in the CT image. In a case in which the CT image is normal (an example of a "first case", Process P2), the processing proceeds to Step S4, and in a case in which the CT image is not normal (Process P3), the processing proceeds to Step S5.

In Step S4, the processor 16B causes the display 20B of the PACS viewer 20 to display the normal CT image in the display form A, and performs post-processing of the processing form A (Process P4). Furthermore, the processor 16B gives accessory information of "type A" to the CT image, stores it in the image storage server 14, and ends the processing of the present flowchart. The post-processing of the processing form A is an example of post-processing in the first case in which it is determined that the medical image is normal in the first determination.

In Step S5 (an example of a "second determination step"), the processor 16B inputs the abnormal CT image to each company's CAD processing server 18. Each company's CAD processing server 18 inputs the CT image to the second determination unit 18A and acquires the second determination result (Process P5).

As illustrated in Fig. 3, the second determination unit 18A includes a lesion detection AI 18B manufactured by Company A that detects a disease α, a lesion detection AI 18C manufactured by Company A that detects a disease β, a lesion detection AI 18D manufactured by Company A that detects a disease γ, a lesion detection AI 18E manufactured by Company B that detects the disease γ, and a lesion detection AI 18F manufactured by Company C that detects the disease β. For example, the disease α is lung cancer, the disease β is pneumonia, and the disease γ is pneumothorax.

Each of the lesion detection AIs 18B to 18F is a trained model (an example of a "second trained model") that outputs a region of a disease (a lesion region, an example of "abnormality") in a CT image in a case in which the CT image of the lung is input, and each includes a convolutional neural network. Each of the lesion detection AIs 18B to 18F is generated by performing deep learning using a label image in which a doctor labels a region of each disease on the CT image of the lung as training data.

Each of the lesion detection AIs 18B to 18F is set to obtain the probability of each disease for each pixel of the CT image, and a pixel exceeding a predetermined threshold value is considered as the region of the disease. Each of the lesion detection AIs 18B to 18F has a higher specificity compared to a case in which lesion detection is performed independently, that is, a relatively high threshold value is set. As a result, each of the lesion detection AIs 18B to 18F detects a location that is more likely to be a lesion. This is because the abnormality is small in a health checkup and the like.

The second determination unit 18A inputs the CT image to each of the lesion detection AIs 18B to 18F. Each of the lesion detection AIs 18B to 18F performs lesion detection processing on the CT image and outputs it as the second determination result.

In Step S6, the processor 16B acquires the second determination result from the second determination unit 18A and integrates the second determination result (Process P6).

In Step S7, the processor 16B determines whether or not an abnormality (here, a lesion) is present in the CT image from the integrated second determination result. In a case in which the abnormality is present in the CT image (an example of a "second case", Process P7), the processing proceeds to Step S8, and in a case in which the abnormality is absent in the CT image (an example of a "third case", Process P8), the processing proceeds to Step S9.

In Step S8, the processor 16B causes the display 20B of the PACS viewer 20 to display the CT image in which the abnormality is present in the display form B, and performs post-processing of the processing form B (Process P9). The post-processing of the processing form B is an example of post-processing in the second case in which it is determined that the medical image is not normal in the first determination and it is determined that the abnormality is present in the second determination. Furthermore, the processor 16B gives accessory information of "type B" to the CT image, stores it in the image storage server 14, and ends the processing of the present flowchart.

On the other hand, in Step S9, the processor 16B causes the display 20B of the PACS viewer 20 to display the CT image in which the abnormality is absent in the display form C, and performs post-processing of the processing form C (Process P10). The post-processing of the processing form C is an example of post-processing in the third case in which it is determined that the medical image is not normal in the first determination and it is determined that the abnormality is absent in the second determination. Furthermore, the processor 16B gives accessory information of "type C" to the CT image, stores it in the image storage server 14, and ends the processing of the present flowchart.

The processor 16B causes the display 20B to display the determination results differently between the display form A, and the display form B and the display form C. That is, the processor 16B causes the display 20B to display the determination results differently between the display form A, and the display form B and the display form C. The processor 16B may causes the display 20B to display the determination result differently between the display form B and the display form C.

For example, in the display form A, the fact that there is a high probability that there is no abnormality is presented, the doctor's confirmation is skipped, and the fact that there is no abnormality is automatically reported to the patient. In addition, in the display form B, the name of the detected lesion and the region thereof are displayed in a visually recognizable manner in the same manner as in general CAD. Furthermore, in the display form C, the doctor is notified that the lesion is not detected but is not clearly normal.

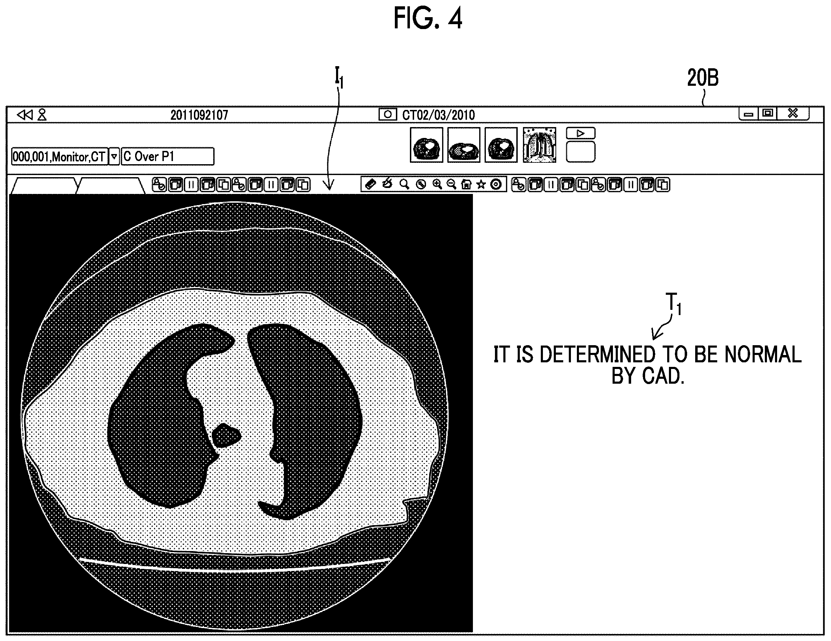

Fig. 4 is a diagram illustrating a display form A. As illustrated in Fig. 4, in the display form A, a CT image I₁ is displayed on the display 20B. In addition, in the display form A, a description T₁ for the CT image I₁ is displayed in a right region of the CT image I₁. Here, the description T₁ "It is determined to be normal by the CAD" is displayed on the display 20B. Since there is a high probability that the CT image I₁ is not abnormal, only a display indicating that the CT image I₁ is normal may be performed without displaying the CT image I₁, and confirmation by the doctor may be skipped.

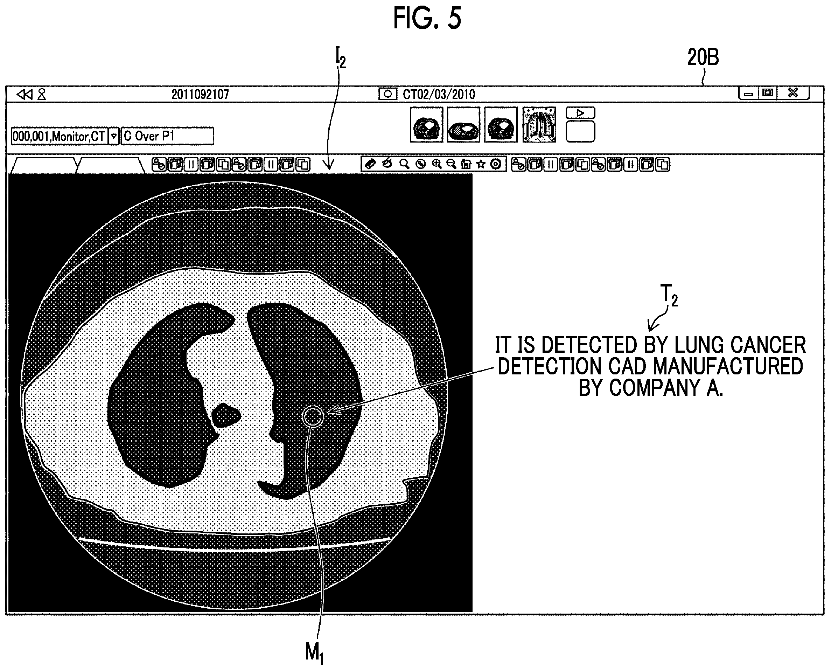

Fig. 5 is a diagram illustrating the display form B. As illustrated in Fig. 5, in the display form B, a CT image I₂ is displayed on the display 20B, and a marker M₁ surrounding a lesion region of the CT image I₂ is superimposed and displayed on the CT image I₂. In addition, in the display form B, a description T₂ for the CT image I₂, which is related to the lesion region surrounded by the marker M₁, is displayed in the right region of the CT image I₂. Here, the lesion region is detected by the lesion detection AI 18B manufactured by Company A that detects the disease α (lung cancer), and a description T₂ "It is detected by lung cancer detection CAD manufactured by Company A." is displayed on the display 20B.

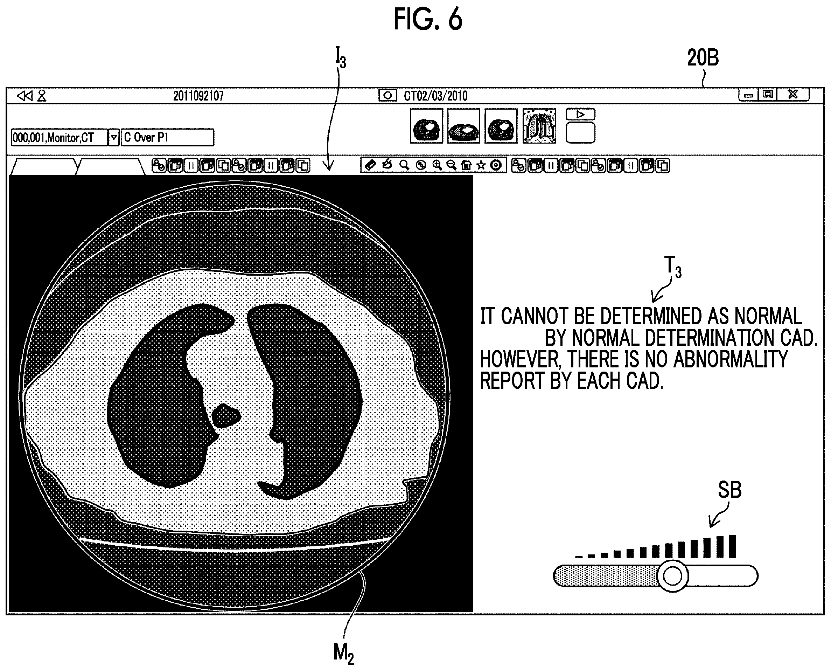

Fig. 6 is a diagram illustrating a display form C. As illustrated in Fig. 6, in the display form C, a CT image I₃ is displayed on the display 20B, and a marker M₂ surrounding the entire CT image I₃ is superimposed and displayed on the CT image I₃. In addition, in the display form C, a description T₃ for the CT image I₃, which is related to the marker M₂, is displayed in the right region of the CT image I₃. Here, the description T₃ "It cannot be determined as normal by the normal determination CAD. However, there is no abnormality report by each CAD." is displayed on the display 20B. In this way, by using a display form different from the display form A and the display form B, it is possible to rectify a determination that is excessively dependent on the lesion detection AI.

In addition, the processor 16B performs the post-processing differently between the processing form A, and the processing form B and the processing form C.

For example, in the processing form A, a flag that indicates that a check by the doctor may be simple is set, and in the processing form B and the processing form C, the flag is not set. In addition, the display order of the interpretation/examination list for confirming the medical image by the doctor may be changed such that the CT image of processing form B and the CT image of the processing form C are given priority over the CT image of processing form A.

In a case of a health checkup, since it is rare to find a disease requiring immediate hospitalization or immediate treatment, it is possible not to notify the result on the spot, or to report the abnormal finding separately after telling the subject that it is normal. Therefore, for the processing form A, it may be reported on the spot as "no abnormality", and then collectively confirm whether or not there is really an abnormality later. For example, as the processing form A, the doctor may perform a simple check at the end of the day. In a case in which an abnormality is found in the simple check, the subject may be separately contacted.

The processor 16B may perform the post-processing differently between the processing form B and the processing form C.

As described above, according to the medical image diagnosis method, the first determination unit 16A can determine whether the medical image is normal. In addition, in a case in which the first determination unit 16A determines that the image is not normal, the second determination unit 18A can determine the presence or absence of the abnormality from the medical image. Therefore, it is possible to reduce the burden on the doctors in a case in which the image diagnosis is performed on a large number of medical images.

### [Others]

For a medical image determined to be normal by the first determination unit 16A, for example, after the closing time of the hospital (an example of a different timing) of the hospital, the second determination unit 18A may perform the third determination processing of determining the presence or absence of an abnormality from the medical image. The third determination processing may be performed in a case in which the medical image is determined to be normal with a probability lower than a predetermined first threshold value in the first determination. The third determination processing is also an example of post-processing in the first case in which it is determined that the medical image is normal in the first determination.

In a case in which it is determined that abnormality is present in the third determination processing, the normal determination AI 16D may be retrained using the training data set of the CT image and a label indicating that it is not normal. Accordingly, the normal determination AI 16D can determine that the CT image is not normal.

In addition, in a case in which it is determined that abnormality is present in the third determination processing, the sensitivity of the normal determination AI 16D may be changed to a value at which the CT image is regarded to be abnormal.

For the medical image in which the second determination unit 18A determines that the abnormality is absent, fourth determination processing of determining the presence or absence of the abnormality from the medical image again by the second determination unit 18A is performed. The fourth determination processing is performed by increasing the sensitivity compared to the second determination processing (reducing the specificity compared to the second determination processing), that is, setting the threshold value to be relatively low, until it is determined that the abnormality is present. The fourth determination processing is also an example of post-processing in the third case in which it is determined that the medical image is not normal in the first determination and it is determined that the abnormality is absent in the second determination.

Accordingly, in the fourth determination processing, a lesion is extracted according to evaluation standards that are acceptable even if something that is not a disease is determined as a disease, and it is reported to the doctor. In a case in which a display form in this case is a display form D, it is desirable to present, to the doctor, that the lesion is extracted by increasing the sensitivity in the display form D.

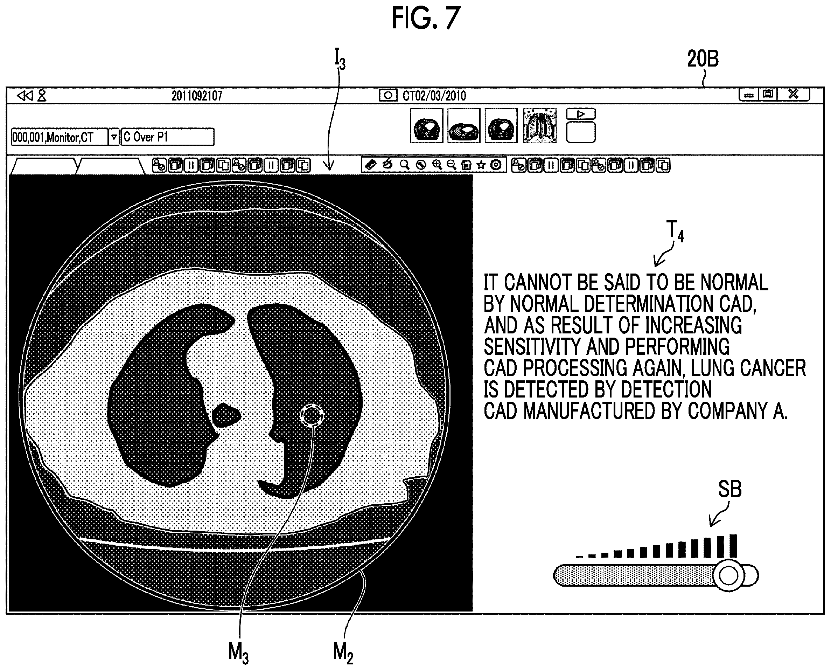

Fig. 7 is a diagram illustrating a display form D. As illustrated in Fig. 7, in the display form D, the CT image I₃ is displayed on the display 20B, the marker M₂ surrounding the entire CT image I₃ is superimposed and displayed on the CT image I₃, and a marker M₃ surrounding the lesion region of the CT image I₃ is further superimposed and displayed on the CT image I₃. Unlike the marker M₁ of the display form B, the marker M₃ is displayed with a broken line to indicate that the lesion is detected by increasing the sensitivity.

In addition, in the display form D, a description T₄ for the CT image I₂, which is related to the marker M₃, is displayed in the right region of the CT image I₂. Here, the description T₄ "It cannot be said to be normal by the normal determination CAD, and as a result of increasing the sensitivity and performing the CAD processing again, a lung cancer is detected by the detection CAD manufactured by Company A." is displayed on the display 20B.

As illustrated in Fig. 6, a slider bar SB for setting the sensitivity of the second determination unit 18A may be displayed in the display form C. In a case in which the user operates the slider bar SB using the input device 20A to increase the sensitivity, the third determination processing may be performed with the set sensitivity, and the display form may proceed to the display form D as illustrated in Fig. 7.

For the medical image in which it is determined that an abnormality is present in the second determination unit 18A, the medical image and the determination result are associated with each other and stored in the image storage server 14 as usual without performing the above-described third and fourth determination processing. This is also an example of post-processing in the second case in which it is determined that the medical image is not normal in the first determination and it is determined that the abnormality is present in the second determination.

In the present embodiment, although the processing with respect to the CT image of the lung has been described as an example, the present invention is not limited thereto. For example, each of the lesion detection AIs of the second determination unit 18A extracts liver cancer, multiple cysts, liver cirrhosis, and fatty liver from the CT image including the liver, and each processing described above may be performed. In addition, each of the above-described processing may be performed on a medical image including other organs.

The technical scope of the present invention is not limited to the scope described in the above-mentioned embodiment. The configurations and the like in each embodiment can be appropriately combined between the respective embodiments without departing from the gist of the present invention.

### Explanation of References

10: medical image diagnosis system
12: modality
14: image storage server
16: result integration CAD processing server
16A: first determination unit
16B: processor
16C: memory
16D: normal determination AI
18: Each company's CAD processing server
18A: second determination unit
18B: lesion detection AI manufactured by Company A
18C: lesion detection AI manufactured by Company A
18D: lesion detection AI manufactured by Company A
18E: lesion detection AI manufactured by Company B
18F: lesion detection AI manufactured by Company C
20: PACS viewer
20A: input device
20B: display
I₁: CT image
I₂: CT image
I₃: CT image
M₁: marker
M₂: marker
M₃: marker
P1 to P10: each process of medical image diagnosis
SB: slider bar
S1 to S9: each step of medical image diagnosis
T₁: description
T₂: description
T₃: description
T₄: description

## Claims

1. A medical image diagnosis system (10) comprising:
at least one processor (16B); and
at least one memory (16C) that stores a command to be executed by the at least one processor,
wherein the at least one processor:
performs (S2) a first determination of determining whether or not a medical image obtained by imaging a subject is normal; and
performs (S5) a second determination of determining the presence or absence of an abnormality from the medical image in a case in which it is determined that the medical image is not normal in the first determination,
**characterised in that** the at least one processor (16B):
performs a further determination of determining the presence or absence of the abnormality from the medical image in a case in which it is determined that the abnormality is absent in the second determination, and
in the further determination, determines the presence or absence of the abnormality from the medical image with a sensitivity relatively higher than a sensitivity in the second determination.

2. The medical image diagnosis system (10) according to claim 1,
wherein the at least one processor (16B) performs a third determination of determining the presence or absence of the abnormality from the medical image in a case in which it is determined that the medical image is normal in the first determination.

3. The medical image diagnosis system (10) according to any claim 2,
wherein the at least one processor (16B) performs the third determination at a timing different from a timing of the second determination.

4. The medical image diagnosis system (10) according to claim 2 or claim 3,
wherein the at least one processor (16B)
performs (S2) the first determination with a probability that the medical image is normal, and
performs the third determination in a case in which the medical image is determined to be normal with a probability lower than a first threshold value in the first determination.

5. The medical image diagnosis system (10) according to any one of claims 2 to 4,
wherein the at least one processor (16B)
performs (S2) the first determination by using a first trained model (16D) that outputs whether or not the medical image is normal in a case in which the medical image is input, and
retrains the first trained model by using a medical image which is determined to have the abnormality in the third determination.

6. The medical image diagnosis system (10) according to claim 5,
wherein the first trained model (16D) is a trained model that has been trained by using combinations of an abnormal medical image, a normal medical image, and labels indicating the presence or absence of the abnormality, as a training data set.

7. The medical image diagnosis system (10) according to any one of claims 1 to 6,
wherein the at least one processor (16B) performs the second determination by using a second trained model that outputs the abnormality of the medical image in a case in which the medical image is input.

8. The medical image diagnosis system (10) according to any one of claims 1 to 7,
wherein, for a first case in which it is determined that the medical image is normal in the first determination, a second case in which it is determined that the medical image is not normal in the first determination and it is determined that the abnormality is present in the second determination, and a third case in which it is determined that the medical image is not normal in the first determination and it is determined that the abnormality is absent in the second determination, the at least one processor displays a determination result of the medical image on a display (20B) differently for the second and third cases than for the first case.

9. The medical image diagnosis system (10) according to claim 8,
wherein the at least one processor (16B) displays the determination result of the medical image on the display (20B) differently between the second case and the third case.

10. The medical image diagnosis system (10) according to claim 8 or claim 9,
wherein the at least one processor (16B) performs different types of post-processing on the medical image for the second and third cases than for the first case.

11. The medical image diagnosis system (10) according to any one of claims 1 to 10,
wherein the at least one processor (16B) performs (S2, S5) the first determination and the second determination for each organ of the subject from the medical image.

12. A medical image diagnosis method comprising:
a first determination step (S2) of determining whether or not a medical image obtained by imaging a subject is normal; and
a second determination step (S5) of determining presence or absence of an abnormality from the medical image in a case in which it is determined that the medical image is not normal in the first determination step,
the method **characterised by** comprising:
a further determination step of determining the presence or absence of the abnormality from the medical image in a case in which it is determined that the abnormality is absent in the second determination step,
wherein the further determination step comprises determining the presence or absence of the abnormality from the medical image with a sensitivity relatively higher than a sensitivity in the second determination step.

13. A non-transitory, computer-readable recording medium which records thereon, a program for causing a computer to execute the medical image diagnosis method according to claim 12.

## Patentansprüche

1. Diagnosesystem (10) für medizinische Bilder, umfassend:
mindestens einen Prozessor (16B); und
mindestens einen Speicher (16C), der einen Befehl, der von dem mindestens einen Prozessor auszuführen ist, speichert,
wobei der mindestens eine Prozessor:
eine erste Bestimmung des Bestimmens, ob ein medizinisches Bild, das durch Abbilden eines Motivs erhalten wird, normal ist oder nicht, durchführt (S2); und
eine zweite Bestimmung des Bestimmens des Vorhandenseins oder Nichtvorhandenseins einer Abnormalität aus dem medizinischen Bild in einem Fall, in dem bestimmt wird, dass das medizinische Bild bei der ersten Bestimmung nicht normal ist, durchführt (S5),
**dadurch gekennzeichnet, dass** der mindestens eine Prozessor (16B):
eine weitere Bestimmung des Bestimmens des Vorhandenseins oder Nichtvorhandenseins der Abnormalität aus dem medizinischen Bild in einem Fall, in dem bestimmt wird, dass die Abnormalität bei der zweiten Bestimmung nicht vorhanden ist, durchführt, und
bei der weiteren Bestimmung das Vorhandensein oder Nichtvorhandensein der Abnormalität aus dem medizinischen Bild mit einer Empfindlichkeit, die relativ höher als eine Empfindlichkeit bei der zweiten Bestimmung ist, bestimmt.

2. Diagnosesystem (10) für medizinische Bilder nach Anspruch 1,
wobei der mindestens eine Prozessor (16B) eine dritte Bestimmung des Bestimmens des Vorhandenseins oder Nichtvorhandenseins der Abnormalität aus dem medizinischen Bild in einem Fall, in dem bestimmt wird, dass das medizinische Bild bei der ersten Bestimmung normal ist, durchführt.

3. Diagnosesystem (10) für medizinische Bilder nach Anspruch 2,
wobei der mindestens eine Prozessor (16B) die dritte Bestimmung zu einem Zeitpunkt, der sich von einem Zeitpunkt der zweiten Bestimmung unterscheidet, durchführt.

4. Diagnosesystem (10) für medizinische Bilder nach Anspruch 2 oder Anspruch 3,
wobei der mindestens eine Prozessor (16B)
die erste Bestimmung mit einer Wahrscheinlichkeit, dass das medizinische Bild normal ist, durchführt (S2), und
die dritte Bestimmung in einem Fall, in dem das medizinische Bild mit einer Wahrscheinlichkeit, die niedriger als ein erster Schwellenwert bei der ersten Bestimmung ist, als normal bestimmt wird, durchführt.

5. Diagnosesystem (10) für medizinische Bilder nach einem der Ansprüche 2 bis 4,
wobei der mindestens eine Prozessor (16B)
die erste Bestimmung durch Verwenden eines ersten trainierten Modells (16D), das in einem Fall, in dem das medizinische Bild eingegeben wird, ausgibt, ob das medizinische Bild normal ist oder nicht, durchführt (S2), und
das erste trainierte Modell durch Verwenden eines medizinischen Bildes, das bestimmt wird, bei der dritten Bestimmung die Abnormalität aufzuweisen, neu trainiert.

6. Diagnosesystem (10) für medizinische Bilder nach Anspruch 5,
wobei das erste trainierte Modell (16D) ein trainiertes Modell ist, das durch Verwenden von Kombinationen eines abnormalen medizinischen Bildes, eines normalen medizinischen Bildes und Etiketten, die das Vorhandensein oder Nichtvorhandensein der Abnormalität angeben, als ein Trainingsdatensatz trainiert worden ist.

7. Diagnosesystem (10) für medizinische Bilder nach einem der Ansprüche 1 bis 6,
wobei der mindestens eine Prozessor (16B) die zweite Bestimmung durch Verwenden eines zweiten trainierten Modells, das in einem Fall, in dem das medizinische Bild eingegeben wird, die Abnormalität des medizinischen Bildes ausgibt, durchführt.

8. Diagnosesystem (10) für medizinische Bilder nach einem der Ansprüche 1 bis 7,
wobei für einen ersten Fall, in dem bei der ersten Bestimmung bestimmt wird, dass das medizinische Bild normal ist, einen zweiten Fall, in dem bei der ersten Bestimmung bestimmt wird, dass das medizinische Bild nicht normal ist, und bei der zweiten Bestimmung bestimmt wird, dass die Abnormalität vorhanden ist, und einen dritten Fall, in dem bei der ersten Bestimmung bestimmt wird, dass das medizinische Bild nicht normal ist, und bei der zweiten Bestimmung bestimmt wird, dass die Abnormalität nicht vorhanden ist, der mindestens eine Prozessor ein Bestimmungsergebnis des medizinischen Bildes auf einer Anzeige (20B) für den zweiten und dritten Fall anders als für den ersten Fall anzeigt.

9. Diagnosesystem (10) für medizinische Bilder nach Anspruch 8,
wobei der mindestens eine Prozessor (16B) das Bestimmungsergebnis des medizinischen Bildes auf der Anzeige (20B) zwischen dem zweiten Fall und dem dritten Fall unterschiedlich anzeigt.

10. Diagnosesystem (10) für medizinische Bilder nach Anspruch 8 oder Anspruch 9,
wobei der mindestens eine Prozessor (16B) für den zweiten und den dritten Fall verschiedene Typen von Nachverarbeitung an dem medizinischen Bild als für den ersten Fall durchführt.

11. Diagnosesystem (10) für medizinische Bilder nach einem der Ansprüche 1 bis 10,
wobei der mindestens eine Prozessor (16B) die erste Bestimmung und die zweite Bestimmung (S2, S5) für jedes Organ der Untersuchungsperson aus dem medizinischen Bild durchführt.

12. Diagnoseverfahren für medizinische Bilder, umfassend:
einen ersten Bestimmungsschritt (S2) des Bestimmens, ob ein medizinisches Bild, das durch Abbilden einer Untersuchungsperson erhalten wird, normal ist oder nicht; und einen zweiten Bestimmungsschritt (S5) des Bestimmens von Vorhandensein oder Nichtvorhandensein einer Abnormalität aus dem medizinischen Bild in einem Fall, in dem bei dem ersten Bestimmungsschritt bestimmt wird, dass das medizinische Bild bei dem ersten Bestimmungsschritt nicht normal ist,
wobei das Verfahren **dadurch gekennzeichnet ist, dass** es umfasst:
einen weiteren Bestimmungsschritt des Bestimmens des Vorhandenseins oder Nichtvorhandenseins der Abnormalität aus dem medizinischen Bild in einem Fall, in dem bei dem zweiten Bestimmungsschritt bestimmt wird, dass die Abnormalität bei dem zweiten Bestimmungsschritt nicht vorhanden ist,
wobei der weitere Bestimmungsschritt Bestimmen des Vorhandenseins oder Nichtvorhandenseins der Abnormalität aus dem medizinischen Bild mit einer Empfindlichkeit, die relativ höher als eine Empfindlichkeit bei dem zweiten Bestimmungsschritt ist, umfasst.

13. Nicht flüchtiges, computerlesbares Aufzeichnungsmedium, das darauf ein Programm aufzeichnet, das einen Computer veranlasst, das Diagnoseverfahren für medizinische Bilder nach Anspruch 12 auszuführen.

## Revendications

1. Système de diagnostic d'images médicales (10) comprenant :
au moins un processeur (16B) ; et
au moins une mémoire (16C) qui stocke une commande à exécuter par l'au moins un processeur,
dans lequel l'au moins un processeur :
effectue (S2) une première détermination consistant à déterminer si une image médicale obtenue par imagerie d'un sujet est normale ou non ; et
effectue (S5) une deuxième détermination consistant à déterminer la présence ou l'absence d'une anomalie à partir de l'image médicale dans un cas où il est déterminé que l'image médicale n'est pas normale lors de la première détermination,
**caractérisé en ce que** l'au moins un processeur (16B) :
effectue une détermination supplémentaire consistant à déterminer la présence ou l'absence de l'anomalie à partir de l'image médicale dans un cas où il est déterminé que l'anomalie est absente lors de la deuxième détermination, et
lors de la détermination supplémentaire, détermine la présence ou l'absence de l'anomalie à partir de l'image médicale avec une sensibilité relativement plus élevée qu'une sensibilité lors de la deuxième détermination.

2. Système de diagnostic d'images médicales (10) selon la revendication 1,
dans lequel l'au moins un processeur (16B) effectue une troisième détermination consistant à déterminer la présence ou l'absence de l'anomalie à partir de l'image médicale dans un cas où il est déterminé que l'image médicale est normale lors de la première détermination.

3. Système de diagnostic d'images médicales (10) selon la revendication 2,
dans lequel l'au moins un processeur (16B) effectue la troisième détermination à un moment différent d'un moment de la deuxième détermination.

4. Système de diagnostic d'images médicales (10) selon la revendication 2 ou la revendication 3,
dans lequel l'au moins un processeur (16B)
effectue (S2) la première détermination avec une probabilité que l'image médicale soit normale, et
effectue la troisième détermination dans un cas où l'image médicale est déterminée comme étant normale avec une probabilité inférieure à une première valeur seuil lors de la première détermination.

5. Système de diagnostic d'images médicales (10) selon l'une quelconque des revendications 2 à 4,
dans lequel l'au moins un processeur (16B)
effectue (S2) la première détermination en utilisant un premier modèle entraîné (16D) qui indique si l'image médicale est normale ou non dans un cas où l'image médicale est entrée, et
entraîne à nouveau le premier modèle entraîné en utilisant une image médicale qui est déterminée comme ayant l'anomalie lors de la troisième détermination.

6. Système de diagnostic d'images médicales (10) selon la revendication 5,
dans lequel le premier modèle entraîné (16D) est un modèle entraîné qui a été entraîné en utilisant des combinaisons d'une image médicale anormale, d'une image médicale normale et d'étiquettes indiquant la présence ou l'absence de l'anomalie, en tant qu'ensemble de données d'entraînement.

7. Système de diagnostic d'images médicales (10) selon l'une quelconque des revendications 1 à 6,
dans lequel l'au moins un processeur (16B) effectue la deuxième détermination en utilisant un deuxième modèle entraîné qui indique l'anomalie de l'image médicale dans un cas où l'image médicale est entrée.

8. Système de diagnostic d'images médicales (10) selon l'une quelconque des revendications 1 à 7,
dans lequel, pour un premier cas où il est déterminé que l'image médicale est normale lors de la première détermination, un deuxième cas où il est déterminé que l'image médicale n'est pas normale lors de la première détermination et il est déterminé que l'anomalie est présente lors de la deuxième détermination, et un troisième cas où il est déterminé que l'image médicale n'est pas normale lors de la première détermination et il est déterminé que l'anomalie est absente lors de la deuxième détermination, l'au moins un processeur affiche un résultat de détermination de l'image médicale sur un affichage (20B) différemment pour les deuxième et troisième cas que pour le premier cas.

9. Système de diagnostic d'images médicales (10) selon la revendication 8,
dans lequel l'au moins un processeur (16B) affiche le résultat de détermination de l'image médicale sur l'affichage (20B) différemment entre le deuxième cas et le troisième cas.

10. Système de diagnostic d'images médicales (10) selon la revendication 8 ou la revendication 9,
dans lequel l'au moins un processeur (16B) effectue différents types de post-traitement sur l'image médicale pour les deuxième et troisième cas que pour le premier cas.

11. Système de diagnostic d'images médicales (10) selon l'une quelconque des revendications 1 à 10,
dans lequel l'au moins un processeur (16B) effectue (S2, S5) la première détermination et la deuxième détermination pour chaque organe du sujet à partir de l'image médicale.

12. Procédé de diagnostic d'images médicales comprenant :
une première étape de détermination (S2) consistant à déterminer si une image médicale obtenue par imagerie d'un sujet est normale ou non ; et
une deuxième étape de détermination (S5) consistant à déterminer la présence ou l'absence d'une anomalie à partir de l'image médicale dans un cas où il est déterminé que l'image médicale n'est pas normale lors de la première étape de détermination,
le procédé **caractérisé en ce qu'**il comprend :
une étape de détermination supplémentaire consistant à déterminer la présence ou l'absence de l'anomalie à partir de l'image médicale dans un cas où il est déterminé que l'anomalie est absente lors de la deuxième étape de détermination,
dans lequel l'étape de détermination supplémentaire comprend la détermination de la présence ou de l'absence de l'anomalie à partir de l'image médicale avec une sensibilité relativement plus élevée qu'une sensibilité lors de la deuxième étape de détermination.

13. Support d'enregistrement non transitoire, lisible par ordinateur, sur lequel est enregistré un programme pour amener un ordinateur à exécuter le procédé de diagnostic d'images médicales selon la revendication 12.
